# EUROPEAN PATENT SPECIFICATION

(11) **EP 2 831 222 B1**
(45) Date of publication and mention of the grant of the patent: **26.12.2018**
(21) Application number: 12872506.6
(22) Date of filing: 27.03.2012
(51) Int. Cl.: C12N 1/00, C12N 5/0793

(54) **NEURONAL CULTURE MEDIUM AND METHOD FOR PRODUCING IN VIVO-LIKE AND ENHANCED SYNAPTOGENESIS NEURON MODEL**
NEURONALES KULTURMEDIUM UND VERFAHREN ZUR ERZEUGUNG EINES IN-VIVO-ÄHNLICHEN UND ERWEITERTEN SYNAPTOGENESE-NEURONEN-MODELLS
MILIEU DE CULTURE NEURONAL ET PROCÉDÉ DE PRODUCTION D'UN MODÈLE DE NEURONE À SYNAPTOGÉNÈSE DE TYPE IN-VIVO ET AMÉLIORÉE

(43) Date of publication of application: 04.02.2015
(73) Proprietor: Okinawa Institute of Science and Technology Graduate University, Okinawa 904-0495 (JP)
(72) Inventor: DIMITROV, Dimitar Ivanov, Kunigami-gun Okinawa 904-0495 (JP); GUILLAUD, Laurent Elie, Kunigami-gun Okinawa 904-0495 (JP); TAOUFIQ, Zacharie Mehdi Francois, Kunigami-gun Okinawa 904-0495 (JP); TAKAHASHI, Tomoyuki, Kunigami-gun Okinawa 904-0495 (JP)
(74) Representative: Herzog, Fiesser & Partner Patentanwälte PartG mbB
(86) International application number: PCT/JP2012/002129
(87) International publication number: WO 2013/144999

(56) References cited:
- WO-A1-94/16059
- WO-A2-03/092716
- WO-A2-2005/007797
- US-A1- 2011 112 034
- DINAH W. Y. SAH ET AL: "Regulation of voltage- and ligand-gated currents in rat hippocampal progenitor cellsin vitro", JOURNAL OF NEUROBIOLOGY, vol. 32, no. 1, 1 January 1997 (1997-01-01), pages 95-110, XP055212108, US ISSN: 0022-3034, DOI: 10.1002/(SICI)1097-4695(199701)32:1<95::AI D-NEU9>3.0.CO;2-9
- L. P. DINIZ ET AL: "Astrocyte-induced Synaptogenesis Is Mediated by Transforming Growth Factor Signaling through Modulation of D-Serine Levels in Cerebral Cortex Neurons", JOURNAL OF BIOLOGICAL CHEMISTRY, vol. 287, no. 49, 30 November 2012 (2012-11-30), pages 41432-41445, XP055212586, ISSN: 0021-9258, DOI: 10.1074/jbc.M112.380824
- J. GERARD G. BORST ET AL: "The Calyx of Held Synapse: From Model Synapse to Auditory Relay", ANNUAL REVIEW OF PHYSIOLOGY, vol. 74, no. 1, 17 March 2012 (2012-03-17) , pages 199-224, XP055212448, ISSN: 0066-4278, DOI: 10.1146/annurev-physiol-020911-153236
- CHRISTIAN LOHMANN ET AL.: 'Development of a Topographically Organized Auditory Network in Slice Culture Is Calcium Dependent' JOURNAL OF NEUROBIOLOGY vol. 34, 1998, pages 97 - 112, XP055169185
- STEFANIE KAECH ET AL.: 'Culturing hippocampal neurons' NATURE PROTOCOLS vol. 1, 2006, pages 2406 - 2415, XP055169186
- HAKAN KUCUKDERELI ET AL.: 'Control of excitatory CNS synaptogenesis by astrocyte-secreted proteins Hevin and SPARC' PROC. NATL. ACAD. SCI. USA vol. 108, no. 32, 2011, pages E440 - E449, XP055169187
- DANIELA H. MAUCH ET AL.: 'CNS Synaptogenesis Promoted by Glia-Derived Cholesterol' SCIENCE vol. 294, no. 5545, 2001, pages 1354 - 1357, XP002234731

## Description

### Technical Field

The present disclosure relates to a supplement that allows neuronal cells (neurons) to reproduce *in vivo* morphological, physiological, and developmental properties *in vitro,* increases the number of synapses formed with neurons and improves the neurotransmitter release, a neuronal culture medium containing the supplement, and a method for producing an *in vivo*-like and enhanced synaptogenesis neuron model using the medium.

### Background Art

Synapses are highly specialized connection between neurons that are the fundamental units of the nervous system. They are generated during prenatal and postnatal development with high precision and fidelity. Functional synapse contacts occur between two neurons (e.g., axosomatic, axodendritic, axoaxonic, and dendrodendritic synapses) in the central nervous system, and between one neuron and its effector cell in the periphery (e.g. the neuromuscular junction synapse between a motorneuron and a muscle cell).

Synapse formation, maturation, and stabilization require tightly regulated molecular communications between pre-, post-synaptic cells and glial cells, to finally permit proper neurotransmitter release. In addition, higher brain functions such as learning, memory, and perception processes, rely on neuronal synapse development and plasticity. Neurons may establish many synaptic connections, organized into networks, and this number of synapses is progressively decreased during normal aging and highly disrupted in neurodegenerative diseases (Non-Patent Literature 1 and 2).

In the formation of synapses and neuronal networks, a crucial role is played by environmental molecular determinants to control the developmental timing, the homing, and the number of synapses. The culture technique of dissociated primary neurons has been a popular tool for more than 40 years. Therefore in recent years, efforts have been made by neuroscience laboratories and pharmaceutical companies in the development of neuronal culture medium *in vitro.* Several primary culture models such as for cortical neurons, hippocampal neurons, and neuromuscular junction were developed (Non-Patent Literatures 3-5). Their development has allowed easy access to individual neurons for electrophysiology, high resolution imaging and pharmacological experiments. Indeed, the study of neurons and neuronal network formation *in vitro* that can reproduce as close as possible the *in vivo* properties is of great interest in deciphering fundamental brain developmental mechanisms. Moreover, finding molecules that modulate the development of neuron networks is an important step toward finding therapies and treatments in neurodegenerative diseases and aging. A giant central nervous system synapse called calyx of Held is being widely used as a model to study synaptic development and function by anatomists and electrophysiologists; the calyx of Held is an axosomatic terminal in the mammalian auditory brainstem (Non-Patent Literatures 6 to 10). However, it has been prohibitively difficult to raise this synapse *in vitro.*

Conventional standard *in vitro* neuronal culture medium includes a commercially available mixture based on Ham F12 and MEM (registered trademark) preconditioned by astrocytes, or medium supplements such as N2 medium (Non-Patent Literature 11), and B-27(registered trademark) supplement (Life Technologies). These supplements can support long term culture of neuron *in vitro.* Moreover, the use of B-27 supplement supports the culture of neuronal cells without an astrocyte feeder layer. However, the main aim of media containing these supplements is to increase neuronal cell viability and longevity, but no successful attempts were made to reproduce *in vivo* morphological, physiological, and developmental properties of synapses or to increase synapse numbers or neurotransmitter release. To date, in soluble molecules, only a few such as thrombospondin, cholesterol and hevin were identified as a clear inducer of the number of synapses (Patent Literature 1, Non-Patent Literatures 12-13).

Against the above backdrop, in order to study the functions of synapses such as the calyx of Held in detail, it is necessary to optimize a cell culture medium for primary neuronal culture by searching for cofactors that allow neurons to reproduce *in vivo* morphological, physiological, and developmental properties in an *in vitro* system. However, methods for producing such neuron model capable of reproducing *in vivo-*like properties of neuron synapses in an *in vitro* system have not been known.

### CITATION LIST

### Patent Literature

PTL 1: US05/024363

### Non-Patent Literature

NPL 1: Selkoe D. J., 2002, Science, 298(5594): 789-91
NPL 2: Lee S. H. and Sheng M., 2000, Curr Opinion in Neurobiol., 10(1):125-31
NPL 3: Murray M.R., 1965, Methods, Biology and Physiology, 2: 373-455
NPL 4: Mains R.E. and Patterson P.H., 1973, J. Cell Biol., 59: 329-45
NPL 5: Dichter M.A., 1978, Brain Res, 149: 279-93
NPL 6: Forsythe I. J., 1994, Physiol, 479: 381-7;
NPL 7: Yamashita et al., 2010, Nat. Neurosci, 13(7):838-44
NPL 8: Micheva K. et al., 2003, Nat. Neurosci., 6(9):925-32
NPL 9: Takahashi T. et al., 1996, Science, 274(5287):594-7
NPL 10: Borst J. and Van Hoeve J., 2012, Annu. Rev. Physiol., 74:199-224
NPL 11: Bottenstein J. E. & Sato G. H., 1979, Proc Natl Acad Sci, 76 (1) 514-7
NPL 12: Mauch D.H., et al., 2001, Science, 294 (5545): 1354-7
NPL 13: Kucukdereli H., et al., 2011, PNAS, 108 (32): E440-9

### SUMMARY OF INVENTION

### Technical Problem

An object of the present invention is to develop and provide a method for producing a neuron model by improving a cell culture medium composition and a cell culture medium composition necessary for the production of such neuron model.

### Solution to Problem

As a result of intensive research and development in order to achieve the above objects, the present inventors cultured primary neurons and the like in a medium comprising a conventional cell culture medium supplemented with specific supplements and thus succeeded in increasing the number of synapses formed with neurons and improving neurotransmitter release over the figures for neurons cultured in a cell culture medium not containing such supplements. Further, the present inventors succeeded in allowing neurons to reproduce *in vivo*-like morphological, physiological, developmental properties *in vitro.* The present invention has been completed based on the above study results. The present invention provides the subject matter defined in the claims; moreover, the following is disclosed.
(1) A medium supplement for producing *in vivo*-like and enhanced synaptogenesis neuron model, which comprises any or any combination of neurotrophin-3 (NT-3), potassium or a salt thereof, and basic fibroblast growth factor (FGF-2).
(2) The medium supplement of (1), wherein the enhanced synaptogenesis comprises an increased number of synapse and an increased neurotransmitter release of synapse.
(3) The medium supplement of (2), wherein the synapse is selected from the group consisting of Calyx of Held synapse, neuronal-neuronal synapse and neuronal-peripheral effector cell synapse.
(4) A medium for producing *in vivo*-like and enhanced synaptogenesis neuron model, which is obtained by adding the medium supplement of any one of (1) to (3) to a cell culture medium.
(5) The medium of (4), wherein the cell culture medium is Eagle MEM medium, DMEM medium, Ham F10 medium, Ham F12 medium, M199 medium, improved high-performance 199 medium, RPMI-1640 medium, DMEM/F12, or Neurobasal medium.
(6) The medium of (4) or (5), which comprises neurotrophin-3 at a final concentration of 1 to 500 ng/mL, potassium or a salt thereof at a final concentration of 1 to 50 mM (milli molar), and/or basic fibroblast growth factor at a final concentration of 1 to 100 ng/mL.
(7) The medium of (4), which comprises neurotrophin-3 at a final concentration of 50 ng/mL, potassium or a salt thereof at a final concentration of 25 mM, and/or basic fibroblast growth factor at a final concentration of 5 ng/mL.
(8) A method for producing an *in vivo*-like and enhanced synaptogenesis neuron model, which comprises a step of culturing cells *in vitro* using the medium of any one of (4) to (7).
(9) The method of (8), wherein the cells are neurons, neural precursor cells, neural stem cells, neuronal cell lines, or neurons in co-culture with their effector cells. F

An *in vivo*-like and enhanced synaptogenesis neuron model can be readily formed *in vitro* simply by adding the medium supplement for producing *in vivo*-like and enhanced synaptogenesis neuron model of the present invention to a known cell culture medium.

The neuron model having reproduced *in vivo* properties can be readily produced using the medium for producing *in vivo*-like and enhanced synaptogenesis neuron model of the present invention and the method for producing an *in vivo*-like and enhanced synaptogenesis neuron model using the medium of the present invention.

### BRIEF DESCRIPTION OF DRAWINGS

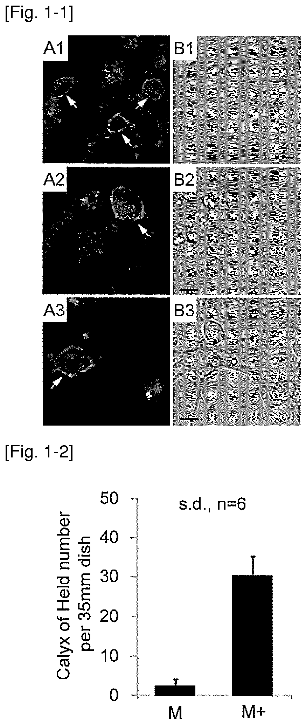

Fig. 1-1 shows the primary cultured calyx of Held (synapse) visualized with FM dye (left column: A1 to A3). Each arrow represents a single Calyx of Held synapse structure in the figure. In addition, differential interference contrast (DIC) microscope images of the synapses of A1 to A3 are shown in B1 to B3 (right column), respectively. The scale bar for the figure is 10 µm.
Fig. 1-2 is a graph showing the number of synapses (each visually confirmed as a calyx of Held) formed during culture for M medium and the same number for the preconditioned medium of the present disclosure, which were obtained via quantification based on the results shown in fig. 1-1. In the figure, "M" denotes M medium and "M+" denotes a preconditioned medium comprising M medium supplemented with the medium supplement of the present disclosure.

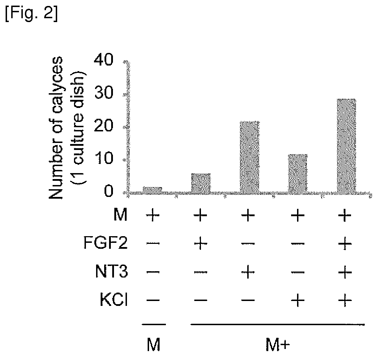

Fig. 2 is a graph showing the number of synapses (each visually confirmed as a calyx of Held) formed during culture for M medium and the same numbers for the preconditioned media supplemented with the different types of medium supplements of the present disclosure, which were obtained via quantification.

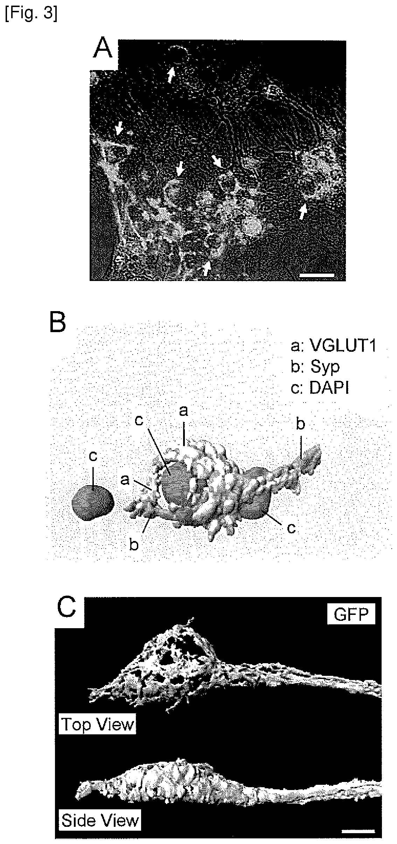

Fig. 3 shows the morphology of a calyx-of-Held-like synapse cultured *in vitro* by the method for producing an *in vivo*-like and enhanced synaptogenesis neuron model of the present invention (A: a confocal image of an FM dye-stained a calyx of Held-like synapse cultured by the production method of the present invention (note that each arrow represents an individual synapse and the scale bar is 20 µm); B: an image of a three-dimensional construct of a fixed immunostained calyx of Held-like synapse on day 15 of culture (DIV 15) (note that VGLUT1 is (a), synaptophysin is (b), and DAPI is (c), and the scale bar is 10 µm); C: an image of a three-dimensional construct of a calyx of Held-like synapse in a living cell expressing GFP and imaged on day 16 of culture (DIV 16)).

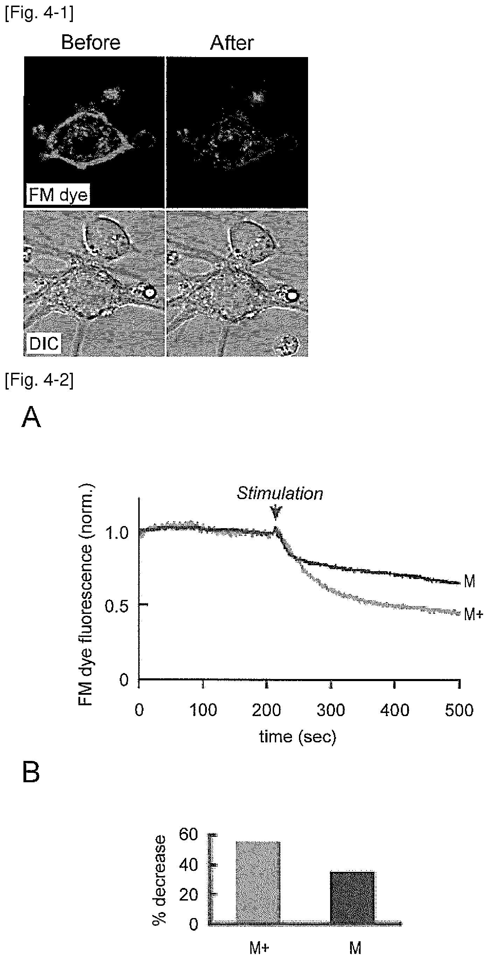

Fig. 4-1 shows FM dye release from calyx of Held-like synapses cultured *in vitro* by the method for producing an *in vivo*-like and enhanced synaptogenesis neuron model of the present invention. The upper panels show FM dye fluorescence images and the lower panels show differential interference contrast (DIC) microscopic images. The panels on the left column show results obtained before stimulation and the panels on the right column show results obtained after stimulation.
Fig. 4-2 shows: (A): FM dye release kinetics for calyx of Held-like synapses cultured *in vitro* by the method for producing an *in vivo*-like and enhanced synaptogenesis neuron model of the present invention; and (B): the FM dye fluorescence decrease rate (%) with respect to the initial fluorescence level upon fluorometry obtained based on the results of A.

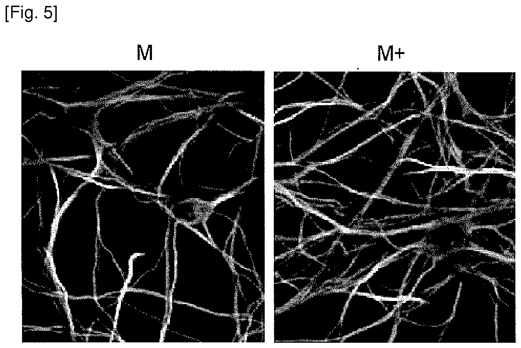

Fig. 5 shows axons and dendritic processes of Hippocampal neurons on day 16 of tubulin-staining culture (DIV 6).

### DESCRIPTION OF EXAMPLES

### Medium supplement for producing in vivo-like and enhanced synaptogenesis neuron model

### 1-1 Outline and definition

A first aspect of the present disclosure relates to a medium supplement for producing *in vivo*-like and enhanced synaptogenesis neuron model (hereinafter some times simply referred to as a "medium supplement"). When the medium supplement of the present disclosure is added to a cell culture medium, the cell culture medium can be used as a medium for producing *in vivo*-like and enhanced synaptogenesis neuron model (hereinafter sometimes referred to as a "preconditioned medium").

Regarding the term *"in vivo*-like and enhanced synaptogenesis neuron model" used herein, *"in vivo*-like neuron model" refers to a neuron that is cultured *in vitro* so as to reproduce *in vivo* developmental, physiological, and morphological cell properties.

Therefore, the *in vivo*-like neuron model can be used in the study of neuroembryology, neurophysiology, and neuronal morphology. Furthermore, "enhanced synaptogenesis neuron model" used herein refers to a neuron, wherein the number of synapses per neuron unit is increased and the neurotransmitter release per synapse is increased over the figures for neurons cultured in a cell culture medium not containing the medium supplement of the present disclosure. The synapse used herein includes Calyx of Held synapse, neuronal-neuronal synapse and neuronal-peripheral effector cell synapse. The term "supplement" used herein simply refers to a supplement in the broadest sense, including the medium supplement of the present disclosure. The term "supplement" means a drug that cannot independently induce cell culture but that can be added to a cell culture medium such that active ingredients of the drug act to impart new properties to the medium. Such supplement comprises, as an active ingredient, any or any combination of a nucleic acid molecule (e.g., a gene, a plasmid containing a gene fragment that can be expressed therein, or siRNA), a peptide (e.g., an oligopeptide or polypeptide), and a low-molecular compound (e.g., a neurotransmitter). Therefore, the medium supplement of the present disclosure can be added to a cell culture medium so as to impart ability to form an *in vivo*-like and enhanced synaptogenesis neuron model to the cell culture medium. The phrase "ability to form an *in vivo*-like and enhanced synaptogenesis neuron model" used herein refers to ability to form a cell into an *in vivo-like* and enhanced synaptogenesis neuron model. In addition, such ability to form an *in vivo*-like and enhanced synaptogenesis neuron model includes ability to enhance synapse formation for increasing the number of synapses to be formed per neuron unit and ability to enhance a neurotransmitter so as to enhance the neurotransmitter release. Therefore, the medium supplement of the present disclosure also can function as a synapse formation enhancer or a neurotransmitter release enhancer.

In addition, a "cell culture medium" is described in detail in the second aspect.

### 1-2.Constitution

The medium supplement of the present disclosure comprises, as an active ingredient that imparts a cell culture medium with ability to form an *in vivo*-like and enhanced synaptogenesis neuron model, neurotrophin-3, potassium or a salt thereof, or basic fibroblast growth factor.

"Neurotrophin-3" (hereinafter abbreviated as "NT-3") is a neurotrophic growth factor. It is a member of the neurotrophin family, which includes NGF, NT-4, NT-5 and BDNF. NT-3 is mainly expressed in the central nervous system and the skeletal muscle, binds to a TrkC tyrosine kinase receptor that is a high-affinity receptor for tyrosine, and activates the signal transduction pathway located downstream of the receptor. Accordingly, NT-3 contributes to, for example, survival and differentiation of existing neurons (Maisonpierre P.C., et al., 1990, Science, 247(4949Pt1): 1446-51; McAllister A.K., et al., 1999, Annu. Rev. Neurosci. 22: 295-318). The origin ofNT-3 of the present invention is not particularly limited. Vertebrate-derived NT-3 is preferable, and mammal-derived NT-3 is more preferable. For instance, if mouse-derived cells are cultured in a preconditioned medium containing the medium supplement of the present invention, NT-3 may be an NT-3 orthologue from a different mammal such as a rat, rabbit, or a human.

The term "potassium" used herein includes potassium ions. In addition, the term "salt thereof' used herein refers to a potassium salt. Examples thereof include potassium chloride, potassium carbonate, potassium acetate, and potassium nitrate. The potassium salt of the medium supplement of the present invention may be a mixture of at least two different potassium salts. In addition, "potassium or a salt thereof' used herein is sometimes simply expressed as "potassium or the like."

The term "basic fibroblast growth factor┘ (bFGF/FGF2) (hereinafter abbreviated as "FGF2") is a protein factor that functions as a cell growth factor, angiogenesis factor, or neurotrohic factor, stimulates proliferation of a variety of cells (e.g., mesenchymal cells, neuroectodermal cells, endothelial cells, and epithelial cells), induces nerve differentiation, survival, and reproduction, and is involved in limb development, angiogenesis, tissue repair, and the like (Reuss B. et al., 2003, Cell Tissue Res., 313: 139-157; Ribatti D. et al., 2007, Cytokine Growth Factor Rev., 18(3-4): 327-34). The origin of FGF2 used in the present invention is not particularly limited. Vertebrate-derived FGF2 is preferable. Mammal-derived FGF2 is more preferable. For example, as in the case of NT-3, it is possible to use an FGF2 orthologue from a mammal of a different species other than the mammal used as the origin of cells cultured in a preconditioned medium using the medium supplement of the present disclosure.

The medium supplement of the present disclosure may contain a combination of at least two members selected from NT-3, potassium or the like, and FGF2. Specifically, a combination of NT-3 and potassium or the like, a combination of NT-3 and FGF2, a combination of potassium or the like and FGF2, and a combination of NT-3, potassium or the like, and FGF2 can be used.

When at least two of the above active ingredients are contained in combination, the active ingredient content is not particularly limited as long as an effective amount of an active ingredient is contained in a preconditioned medium for producing *in vivo*-like and enhanced synaptogenesis neuron model described below supplemented with the medium supplement of the present disclosure. The term "effective amount" used herein refers to the amount of an active ingredient such as NT-3, potassium or the like, or FGF2, which is necessary for allowing the active ingredient to function as expected and causes no or substantially no cytotoxicity to culture cells. For example, as described below, when the medium supplement of the present disclosure is added to a cell culture medium, the cell culture medium preferably contains NT-3, potassium or the like, and FGF2 at final concentrations of 1 to 500 ng/mL, 1 to 50 mM, and 1 to 100 ng/mL, respectively.

The medium supplement of the present disclosure may contain other components unless the above active ingredients are inhibited or deactivated. According to the present invention, the other components include other active ingredients. The phrase "other active ingredients" indicates components that impart effects that differ from the effects imparted by the above active ingredients. Specific examples of such components include BDNF and NGF.

The formulation of the medium supplement of the present disclosure is not particularly limited as long as the active ingredients are not (or are unlikely to be) inhibited or deactivated and readily stored or added to a cell culture medium. Examples of general formulations include liquid and solid (e.g., powder or granule) formulations. Among the active ingredients, NT-3 and FGF2 are proteins and thus they are preferably formulated in a manner such that they are unlikely to be susceptible to heat or degradation enzymes such as protease. For instance, if NT-3 or FGF2 is formulated into a liquid, the liquid may contain a protease inhibitor or the like.

### 1-3.Effects

The addition of the medium supplement of the present disclosure to a cell culture medium makes it possible to impart the ability to form an *in vivo*-like and enhanced synaptogenesis neuron model to the cell culture medium.

### 2.Medium for producing in vivo-like and enhanced synaptogenesis neuron model 2-1. Outline

A second aspect of the present disclosure relates to a medium for producing *in vivo*-like and enhanced synaptogenesis neuron model. The preconditioned medium of the present disclosure is a cell culture medium supplemented with the medium supplement used in the first aspect. Cultured cells can be formed into *in vivo*-like and enhanced synaptogenesis neuron models in the medium.

### 2-2. Constitution

The preconditioned medium of the present disclosure is obtained by adding the medium supplement used in the first aspect to a cell culture medium.

The term "cell culture medium" used in the present invention refers to a known medium that is generally used in the art for cell culture. Any standard cell culture medium or special cell culture medium may be used. The term "standard cell culture medium" used herein refers to a highly versatile standard medium mainly used for culture of a variety of mammal-derived cells. Specific examples of such medium include Eagle MEM (Eagle Minimum Essential Medium), DMEM (Dulbecco's Modified Eagle Medium), Ham F10 (Ham's Nutrient Mixture F10) medium, Ham F12 (Ham's Nutrient Mixture F12) medium, M199 medium, improved high-performance 199 medium (High Performance Medium 199), RPMI-1640 (Roswell Park Memorial Institute-1640) medium, DMEM/F12 (Dulbecco's Modified Eagle Medium/Ham's Nutrient Mixture F12) medium, and Neurobasal medium. In the case of DMEM/F12 medium, the mixture ratio of DMEM to F12 is not particularly limited. Preferably, DMEM and F12 are mixed at a ratio of 6:4 to 4:6 in terms of component weight concentration. The specific composition of such standard cell culture medium is known in the art. Therefore, a medium may be prepared according to the compositions described in appropriate references (e.g., Kaech S. and Banker G., 2006, Nat. Protoc., 1(5): 2406-15) or commercially available products from manufacturers (e.g., life Technologies, Thermo scientific, and Wako Pure Chemical Industries) may be used. In addition, the term "special cell culture medium" used herein refers to an optimized medium for neuron culture comprising a standard cell culture medium supplemented with various supplements or a preconditioned medium capable of inducing cells to differentiate into neurons. Examples thereof include commercially available neuronal culture media produced by manufacturers. A specific example thereof is "M medium" obtained by adding, as supplements, NGF2.5S and BDNF to a neuronal culture medium (Sumitomo Bakelite) that has been prepared by adding a culture supernatant of primary astroglia cells cultured on a nutritive medium and serum albumin to a standard medium prepared by adding insulin and transferrin to DMEM/F12 (5:5).

The content of the medium supplement used in the first aspect that is added to the above cell culture medium is not particularly limited. Preferably, the medium supplement contains NT-3, potassium or the like, and FGF2 at final concentrations of 1 to 500 ng/mL, 1 to 50 mM, and 1 to 100 ng/mL, respectively. More preferably, the medium supplement contains NT-3, potassium or the like, and FGF2 at final concentrations of 10 to 200 ng/mL, 10 to 30 mM, and 3 to 8 ng/mL, respectively. Further preferably, the medium supplement contains NT-3, potassium or the like, and FGF2 at final concentrations of 50 ng/mL, 25 mM, and 5 ng/mL, respectively.

The preconditioned medium of the present disclosure may contain serum according to need. The origin of serum may be any species of organism. However, it is preferably a bird or a mammal. For example, fetal bovine serum (FBS) is preferably used. The final concentration of the serum is preferably 1% to 20%, more preferably 5% to 15%, further preferably 8% to 12%, and particularly preferably 10%. In addition, the medium of the present disclosure may contain, if necessary, a culture supernatant of glia cells (such as astroglia cells) or serum albumin.

### 2-3. Effects

The use of the preconditioned medium of the present disclosure makes it possible to impart the properties described below to cultured cells.

### (1) In-vivo-like structural configuration

Configurations similar to those generally observed under *in vivo* conditions can be reproduced.

### (2) Increase of the number of formed synapses

The number of synapses that can be formed is at least 10 times the number of synapses formed with neurons cultured in a conventional cell culture medium.

### (3) Improvement of neurotransmitter release and functionality of in vivo-like presynaptic cells

Neurotransmitter release from synapses can be improved to a greater extent than is possible with synapses formed with neurons cultured in a conventional cell culture medium. In addition, the intensity and kinetics of release from synapses can be reproduced so as to resemble those observed *in vivo* as closely as possible.

Therefore, neurons that reproduce *in vivo* developmental, physiological, and morphological properties that resemble those observed *in vivo* as closely as possible, which have been difficult to reproduce using a conventional cell culture, can be cultured in a cell culture system using the conditioned medium of the present disclosure.

### 3. Method for producing an in vivo-like and enhanced synaptogenesis neuron model

### 3-1. Outline

An aspect of the present invention relates to a method for producing an *in vivo*-like and enhanced synaptogenesis neuron model according to claim 1. According to the method for producing an *in vivo* neuron model of the present invention, an *in vivo*-like and enhanced synaptogenesis neuron model formed with neurons capable of reproducing *in vivo* developmental, physiological, and morphological properties that resemble those observed *in vivo,* which have been difficult to reproduce using a conventional cell culture medium, can be readily produced by carrying out cell culture using the conditioned medium disclosed herein above.

### 3-2. Configuration

The method for producing an *in vivo*-like and enhanced synaptogenesis neuron model of the present invention comprises the culture step as an essential step. The culture step is specifically described below.

### Culture step

The "culture step" comprises culturing cells *in vitro* using the medium for producing *in vivo*-like and enhanced synaptogenesis neuron model described herein above.

Cells used in this step include all types of cells having the ability to differentiate into neurons. For example, non-neuron-derived cells such as rat adrenal-medulla-derived PC-12 cells are also included. Preferably, neurons (e.g., cerebral neurons and peripheral neurons), neural precursor cells, and neural stem cells are used. Alternatively, a mixture of a plurality of types of cells may be used. In addition, cells that form large-sized synapses are preferable for studies of synapse formation and cell morphology.

For example, ventral cochlear nucleus (VCN) cells and cells of the medial nucleus of the trapezoid body (MNTB) are preferable because they form a gigantic synapse called "calyx of Held" therebetween.

In addition, cells used in the production method of the present invention may be obtained from normal cells or mutant cells. For instance, in order to study cancer cell behavior, cells from the central nervous system cancer (or tumor) may be used. Mutant cells used for the formation of a desired *in vivo*-like and enhanced synaptogenesis neuron model may be selected in an adequate manner depending on purposes of use.

Further, cells used in this step include any type of cultured cells. For example, primary culture cells and cells of established cell lines are included. Examples of primary culture cells include cells separated from the cerebral hemisphere, cerebral cortex, and cranial nerve tissue such as hippocampal or strial tissue. Specific examples of cells of established cell lines include cells established from central nervous system tumors and neuroblastoma.

A medium used in this step is any preconditioned medium described herein above. A preconditioned medium having composition identical thereto may be used during culture. Alternatively, a medium having a different composition may be used upon medium replacement. For example, it is possible to use a preconditioned medium to which a medium supplement containing FGF2 and potassium or the like as active ingredients has been added for primary culture and then to use a preconditioned medium of the same type to which a medium supplement containing FGF2, potassium or the like, and NT-3 as active ingredients has been added for medium replacement.

In principle, a method for culturing cells *in vitro* may be carried out according to a neuron culture method known in the art in this step depending on type and origin of cells to be cultured. In general, culture is carried out under 5% CO₂ at 37 degrees C after sowing of cells on a cell culture medium. More specifically, known culture methods (e.g., Kaech S. and Banker G., 2006, Nat. Protoc. 1(5): 2406-15 for hippocampal neurons; Hilgenberg L.G. and Smith M.A., 2007, J. Vis. Exp., 10: 562 for cortical neurons) can be referred to. In addition, if a preconditioned medium contains no serum, serum can be added at a final concentration of 1% to 20%, preferably 5% to 15%, more preferably 8% to 12%, and further preferably 10% according to need. Further, it is also possible to add a glia cell culture supernatant to the medium of the present disclosure according to need.

The culture period can be adequately determined depending on the type and origin of cells to be cultured and the purposes of culture. For instance, if an *in vivo*-like and enhanced synaptogenesis neuron model that reproduces developmental properties is produced using primary culture cells, culture can be carried out for about 5 to 35 days. If an *in vivo*-like and enhanced synaptogenesis neuron model that reproduces physiological properties is produced using primary culture cells, culture can be carried out for about 5 to 35 days. In addition, if an *in vivo*-like and enhanced synaptogenesis neuron model that reproduces morphological properties is produced using primary culture cells, culture can be carried out for about 5 to 35 days.

### 3-3. Effects

According to the method for producing an *in vivo*-like and enhanced synaptogenesis neuron model of the present invention, neurons capable of reproducing *in vivo* developmental, physiological, and morphological properties can be produced in an *in vitro* cell culture system. Therefore, highly reliable experimental results reflecting *in vivo* conditions can be obtained and new findings about neurons that have been impossible to demonstrate *in vivo* can be discovered by making use of neurons produced by the production method of the present invention in the study of neuroembryology, neurophysiology, and neuronal morphology.

### EXAMPLES

The present invention is specifically described below in greater detail with reference to the following examples. However, the following examples merely describe specific embodiments of the present invention. Therefore, the technical scope of the present invention is not limited thereto.

### Example 1

### Example 1: Confirmation of the ability to enhance synapse formation in an in vivo-like and enhanced synaptogenesis neuron model (1)

### (Object)

The object of this Example is to confirm that an *in vivo*-like and enhanced synaptogenesis neuron model with an increased number of formed synapses can be produced by the method for producing an *in vivo*-like and enhanced synaptogenesis neuron model using the preconditioned medium of the present invention.

### (Method)

### (1) Neuron formation and culture

Before dissection, 35 mm culture dishes (µ-dish^{35mm,low}; Ibidi) were coated with 100 µg/ml poly-D-lysine (Millipore) for 1 hour and washed 3 times with ultrapure water (Millipore). After the wash, the dishes were left for the duration of the dissection/dissociation procedure (2-3 hours) with an open lid under clean bench air flow to dry out.

All experiments described herein were performed in accordance with the guidelines of the Physiological Society of Japan and the guidelines of the Okinawa Institute of Science and Technology Graduate University, and animal experiment regulations at the Okinawa Institute of Science and Technology Graduate University.

B6 or ICR mouse line pups aged E18 to PI were decapitated and their brains extracted. The brains were turn upside-down, and the dural layer stripped aside under a dissecting microscope. For extraction of ventral cochlear nucleus (VCN) neurons, the cochlear nucleus regions were dissected using micro-dissection tools (Feather, Japan). The cochlear nucleus regions were clearly visible under the microscope as sticking out of the rest of the brainstem and stored in a first 15-ml tube containing 3 mL of Hank's balanced salt solution (HBSS) (Life technologies). Next, for extraction of principal neurons of the medial nucleus of the trapezoid body (MNTB), the MNTB region was dissected by making two lateral incisions at the location of the lateral superior olive (LSO) approximately 1 mm from the midline on both sides. Then, a transverse incision was made approximately 2 mm from the pontine nuclei to connect the two lateral incisions. The resulting piece of tissue was scooped using micro-dissection tools (Feather, Japan) at approximately 2 mm depth and put in a second 15-ml tube containing 3 mL of HBSS.

Dissected tissue from both the VCN and MNTB regions was separately dissociated using the Nerve Cell Dissociation Medium (Sumitomo Bakelite) following the manufacturer's instructions. Subsequently, dissociated neurons were counted and plated as an equal mix of VCN neurons and MNTB neurons at a total density 120,000 cells per one dish on the above dish coated with poly-D-lysine. Culture was carried out under 5% CO₂ at 37 degrees C for up to 35 days using M medium obtained by adding 100 ng/mL NGF 2.5S (Life technologies) and 25 ng/mL human BDNF (R&D Systems) to the neuronal culture medium (Sumitomo Bakelite) containing an astroglia cell culture supernatant and the like and the preconditioned medium of the present disclosure obtained by further adding the medium supplement of the present disclosure comprising human FGF2 (Peprotech) (final concentration: 5 ng/mL) and KCl (Nacalai Tesque) (final concentration: 20 mM) to such M medium. At DIV 4, half of the medium was replaced with the conditioned medium of the present disclosure prepared by adding the medium supplement of the present disclosure comprising human FGF2 (Peprotech) (final concentration: 5 ng/mL), KCl (Nacalai Tesque) (final concentration: 20 mM), and human NT-3 (Peprotech) (final concentration: 50 ng/mL) to the M medium. At DIV 8, half of the medium was replaced with the preconditioned medium of the present disclosure prepared by further adding AraC (final concentration: 20 µM), which is an arabinose-operon-modulating protein, to the medium used for medium replacement at DIV 4. At DIV 12, half of the medium was replaced with the preconditioned medium of the present disclosure having the same composition as the medium used for medium replacement at DIV 4.

### (2) Observation of neurons

Cells were stained with 2 µM of FM dye for 1 minute, stimulated with 90 mM of KCl for 2 minutes, let recovered in low KCl (6 mM) with 2 µM of FM dye for 5 minutes. Ten minutes before observation, cells were washed with normal saline. All solutions contained 10 µM of CNQX and 50 µM of APV. Cells were observed using a confocal microscope (LSM 710, Zeiss). Thereafter, formed synapses were counted.

### (Results)

Figs. 1-1 and 1-2 show the results. Based on fig. 1-1, it is understood that Calyx of Held synapse, as having a calyx shape and forming a large synapse structure (around 20 µm large) can be visually identified and counted by direct microscope observation.

Fig. 1-2 shows that the average number of synapses (each confirmed as a calyx of Held) formed in the medium of the present disclosure (corresponding to M+ medium used herein) was found to be 30.3, showing an increase of at least 10-fold over the number of synapses (each confirmed as a calyx of Held) formed during culture in the M medium (average: 2.4). Therefore, it was demonstrated that the medium of the present disclosure supplemented with the medium supplement containing NT-3, potassium or the like, and FGF2 has ability to enhance neuron synapse formation.

### Example 2

### Example 2: Confirmation of the ability to enhance synapse formation in an in vivo-like and enhanced synaptogenesis neuron model (2)

### (Object)

The object of this Example is to confirm that an *in vivo*-like and enhanced synaptogenesis neuron model with an increased number of formed synapses can be produced by the method for producing an *in vivo*-like and enhanced synaptogenesis neuron model even with the use of different preconditioned media supplemented with the medium supplements of the present disclosure separately containing NT-3, a potassium salt (KCL), and FGF2 as active ingredients.

### (Method)

### (1) Neuron formation and culture

Basically, the method used herein was carried out in the manner described in Example 1. In this Example, however, culture was carried out using the following media under 5% CO₂ at 37 degrees C for up to 35 days: M medium; the preconditioned medium of the present disclosure supplemented with the medium supplement of the present disclosure consisting of 5 ng/mL human FGF2 (Peprotech) (M+FGF2 medium); the preconditioned medium of the present disclosure obtained by adding the medium supplement of the present disclosure consisting of KCl (Nacalai Tesque) (final concentration: 25 mM) to M medium (M+KCl medium); the preconditioned medium of the present disclosure obtained by adding the medium supplement of the present disclosure consisting of human NT-3 (Peprotech) (final concentration: 50 ng/mL) to M medium (M+NT-3 medium); and the preconditioned medium of the present disclosure supplemented with the medium supplement of the present disclosure containing human FGF2 (Peprotech) (final concentration: 5 ng/mL), KCl (Nacalai Tesque) (final concentration: 25 mM), and human NT-3 (Peprotech) (final concentration: 50 ng/mL) (M+ medium). During the culture period, half of the medium was replaced at DIV 4, DIV 8, and DIV 12.

### (2) Observation and quantification of neurons

As in the case of Example 1, cultured cells were stained with 2 µM of FM dye for 1 minute, stimulated with 90 mM of KCl for 2 minutes, let recovered in low KCl (6 mM) with 2 µM of FM dye for 5 minutes. Ten minutes before observation, cells were washed with normal saline. All solutions contained 10 µM of CNQX and 50 µM of APV. Cells were observed using a confocal microscope (LSM 710, Zeiss) and the formed synapses were counted. Based on the results, the number of synapses formed by the method for producing an *in vivo*-like and enhanced synaptogenesis neuron model was quantified for each medium supplement used above.

### (Results)

Fig. 2 shows the results. As shown in the figure, it was demonstrated that the numbers of synapses formed not only in the M+FGF2+NT-3+KCl medium but also in the M+FGF2 medium, the M+NT-3 medium, and the M+KCl medium were significantly higher than the number of synapses formed during culture in the M medium.

Therefore, it was demonstrated that NT-3, KCl, and FGF2, which are the active ingredients of the medium supplement of the present disclosure, can independently impart the ability to enhance neuron synapse formation to the preconditioned medium of the present disclosure.

### Example 3

### Example 3: Confirmation of reproducibility of developmental and morphological properties of neurons in an in vivo-like and enhanced synaptogenesis neuron model

### (Object)

The object of this Example is to confirm that the *in vivo*-like and enhanced synaptogenesis neuron model produced by the *in vivo*-like and enhanced synaptogenesis neuron model production method of the present disclosure can reproduce original *in vivo* properties of neurons.

### (Method)

Dissected tissue from both the VCN and MNTB regions was separately dissociated using the Nerve Cell Dissociation Medium (Sumitomo Bakelite) following the manufacturer's instructions. Subsequently, dissociated neurons were counted and plated as an equal mix of VCN neurons and MNTB neurons at a total density 120,000 cells per one dish on the above dish coated with poly-D-lysine. Culture was carried out under 5% CO₂ at 37 degrees C for up to 35 days using M medium obtained by adding 100 ng/mL NGF 2.5S (Life technologies) and 25 ng/mL human BDNF (R&D Systems) to the neuronal culture medium (Sumitomo Bakelite) containing an astroglia cell culture supernatant and the like and the preconditioned medium of the present disclosure obtained by further adding the medium supplement of the present disclosure comprising human FGF2 (Peprotech) (final concentration: 5 ng/mL) and KCl (Nacalai Tesque) (final concentration: 20 mM) to such M medium. At DIV 4, half of the medium was replaced with the conditioned medium of the present disclosure prepared by adding the medium supplement of the present disclosure comprising human FGF2 (Peprotech) (final concentration: 5 ng/mL), KCl (Nacalai Tesque) (final concentration: 20 mM), and human NT-3 (Peprotech) (final concentration: 50 ng/mL) to the M medium. At DIV 8, half of the medium was replaced with the preconditioned medium of the present disclosure prepared by further adding AraC (final concentration: 20 µM), which is an arabinose-operon- modulating protein, to the medium used for medium replacement at DIV 4. At DIV 12, half of the medium was replaced with the preconditioned medium of the present disclosure having the same composition as the medium used for medium replacement at DIV 4. Cell were then fixed in PBS with PFA 4% at room temperature for 10 minutes, permeabilized in 0.2% saponin at room temperature for 30 minutes. Cells were blocked with PBS 3% BSA for 30 minutes, before incubation with primary antibodies (anti-VGLUT1 from Millipore, anti-synaptophysin from Zymed) and further incubation with corresponding secondary antibodies Alexa 488 or 568 (Invitrogen). Cells were mounted with Prolong Gold (Invitrogen) containing DAPI. Images were acquired with Zen software (Zeiss) and 3D reconstruction were made with Emaris (Bitplane).

### (Results)

Fig. 3 shows the results. Based on the results shown in panels B and C, it was found that the morphology of a calyx-of-Held-like synapse in the *in vivo* neuron model produced by the production method of the present invention was reproduced with high similarity to that of a calyx of Held originally observed *in vivo.* Therefore, it was demonstrated that an *in vivo*-like and enhanced synaptogenesis neuron model formed in the preconditioned medium of the present disclosure can reproduce *in vivo* morphological properties of neurons.

### Example 4

### Example 4: Increase of the neurotransmitter release and functionality of presynaptic cells

### (Object)

The object of this Example is to confirm that the *in vivo*-like and enhanced synaptogenesis neuron model of the present invention has the ability to enhance neurotransmitter release with the use of FM dye.

### (Method)

Dissected tissue from both the VCN and MNTB regions was separately dissociated using the Nerve Cell Dissociation Medium (Sumitomo Bakelite) following the manufacturer's instructions. Subsequently, dissociated neurons were counted and plated as an equal mix of VCN neurons and MNTB neurons at a total density 120,000 cells per one dish on the above dish coated with poly-D-lysine. Culture was carried out under 5% CO₂ at 37 degrees C for up to 35 days using M medium obtained by adding 100 ng/mL NGF 2.5S (Life technologies) and 25 ng/mL human BDNF (R&D Systems) to the neuronal culture medium (Sumitomo Bakelite) containing an astroglia cell culture supernatant and the like and the preconditioned medium of the present disclosure obtained by further adding the medium supplement of the present disclosure comprising human FGF2 (Peprotech) (final concentration: 5 ng/mL) and KCl (Nacalai Tesque) (final concentration: 20 mM) to such M medium. At DIV 4, half of the medium was replaced with the conditioned medium of the present disclosure prepared by adding the medium supplement of the present disclosure comprising human FGF2 (Peprotech) (final concentration: 5 ng/mL), KCl (Nacalai Tesque) (final concentration: 20 mM), and human NT-3 (Peprotech) (final concentration: 50 ng/mL) to the M medium. At DIV 8, half of the medium was replaced with the preconditioned medium of the present disclosure prepared by further adding AraC (final concentration: 20 µM), which is an arabinose-operon- modulating protein, to the medium used for medium replacement at DIV 4. At DIV 12, half of the medium was replaced with the preconditioned medium of the present disclosure having the same composition as the medium used for medium replacement at DIV 4. The cultured cells were stained with 2 µM of FM dye for 1 minute, stimulated with 90 mM of KCl for 2 minutes, let recovered in low KCl (6 mM) with 2 µM of FM dye for 5 minutes. Ten minutes before observation, cells were washed with normal saline. All solutions contained 10 µM of CNQX and 50 µM of APV. A time-series observation was performed using a confocal microscope (LSM 710, Zeiss) and a release of synaptic vesicles was induced by a second addition of 90mM KCl for 2 minutes.

### (Results)

Figs. 4-1 and 4-2 show the results. As shown in the figures, it was revealed that the stimulation-induced release of FM dye from the calyx-of-Held-like synapse determined for the *in vivo*-like and enhanced synaptogenesis neuron model (from VCN cells and MNTB cells) of the present invention is about 60% higher than that determined for the neuron model (from VCN cells and MNTB cells) cultured under the same conditions using a cell culture medium. This indicates that the intensity and kinetics of neurotransmitter release from synapses can be reproduced using the *in vivo-*like and enhanced synaptogenesis neuron model of the present invention with high similarity to those observed *in vivo* (figure 3 from Kay A.R. et al., 1999, Neuron, 24(4): 809-17). Therefore, it was demonstrated that an *in vivo*-like and enhanced synaptogenesis neuron model formed using the preconditioned medium of the present disclosure has the ability to enhance neurotransmitter release from neuron synapses.

### Example 5

### Example 5: Enhancement of neural network in an in vivo-like and enhanced synaptogenesis neuron model

### (Object)

The object of this Example is to confirm that the *in vivo*-like and enhanced synaptogenesis neuron model of the present invention results in an enhanced neural network.

### (Method)

Primary hippocampal neurons were obtained as described elsewhere (Kaech S and Banker G, 2006, Nat. Protoc., 1(5): 2406-15). Tubulin cell tracker (Invitrogen) was used onto hippocampal neurons cell culture according to manufacturer instructions. Cells were observed and images were obtained using a confocal microscope (LSM 710, Zeiss)

### (Results)

Fig. 5 shows the results. As shown in the figure, it was demonstrated that an *in vivo-*like and enhanced synaptogenesis neuron model produced by the production method of the present invention using the preconditioned medium of the present disclosure results in a neural network enhanced to a greater extent than is possible with a neuron model formed in a standard cell culture.

### INDUSTRIAL APPLICABILITY

The medium supplement and the preconditioned medium of the present disclosure may be sold to researchers at public or private institutes, universities, hospitals, and private companies such as pharmaceutical companies mainly for neuroscience research purposes. In particular, pharmaceutical companies can use the medium supplement and the preconditioned medium of the present disclosure for high-throughput drug screening tests, as pharmaceutical companies routinely use neuronal cultures to test, for example, new psychotropic drugs for efficacy and safety or to model neurodegenerative diseases. In addition, the medium supplement and the conditioned medium of the present disclosure may be used to improve the *ex vivo* expanded neural stem cell cultures and tissues containing neurons destined for xenograft, allograft, and autograft, and nervous tissue reconstructions procedures in the biomedical engineering industry.
Further, the medium supplement and the preconditioned medium of the present disclosure may be used in the bionics industry (i.e. bridge between biology and microelectronics) and the nanotechnology industry to improve neurons and synapse networks that are raised on silicon chips and brain-computer interface devices.

## Claims

1. A method for producing an *in vivo*-like and enhanced synaptogenesis neuron model, which comprises a step of culturing primary neurons *in vitro* using a medium,
wherein the medium is obtained by adding a supplement to a neuronal culture medium, wherein the supplement comprises a combination of neurotrophin-3, potassium or a salt thereof, and basic fibroblast growth factor, and
wherein in said enhanced synaptogenesis neuron model the number of synapses per neuron unit is increased and the neurotransmitter release per synapse is increased over the figures for the primary neurons cultured in said neuronal culture medium not containing said supplement.

2. The method of claim 1, wherein the *in vivo*-like and enhanced synaptogenesis neuron model is the calyx of Held-like synapse, when the mixture of primary neurons from ventral cochlear nucleus and medial nucleus of the trapezoid body are cultured as the primary neurons.

3. The method of claim 1 or 2, wherein the neuronal culture medium comprises neurotrophin-3 at a final concentration of I to 500 ng/mL, potassium or a salt thereof at a final concentration of 1 to 50 mM, and/or basic fibroblast growth factor at a final concentration of 1 to 100 ng/mL.

4. The method of claim 3, wherein the neuronal culture medium comprises neurotrophin-3 at a final concentration of 50 ng/mL, potassium or a salt thereof at a final concentration of 25 mM, and/or basic fibroblast growth factor at a final concentration of 5 ng/mL.

5. An *in vivo*-like and enhanced synaptogenesis neuron model comprising primary neurons cultured *in vitro* in a medium,
wherein the medium is obtained by adding a supplement to a neuronal culture medium,
wherein the supplement comprises a combination of neurotrophin-3, potassium or a salt thereof; and basic fibroblast growth factor, and
wherein in said enhanced synaptogenesis neuron model the number of synapses per neuron unit is increased and the neurotransmitter release per synapse is increased over the figures for the primary neurons cultured in said neuronal culture medium not containing said supplement.

6. The *in vivo*-like and enhanced synaptogenesis neuron model of claim 5, wherein the neuronal culture medium comprises neurotrophin-3 at a final concentration of 1 to 500 ng/mL, potassium or a salt thereof at a final concentration of 1 to 50 mM, and/or basic fibroblast growth factor at a final concentration of 1 to 100 ng/mL.

7. The in *vivo*-like and enhanced synaptogenesis neuron model of claim 6, wherein the neuronal culture medium comprises neurotrophin-3 at a final concentration of 50 ng/mL, potassium or a salt thereof at a final concentration of 25 mM, and/or basic fibroblast growth factor at a final concentration of 5 ng/mL.

8. Use of a medium supplement or a medium comprising said medium supplement for producing an *in vivo*-like and enhanced synaptogenesis neuron model in primary neuronal cell culture,
wherein the medium supplement comprises a combination of neurotrophin-3, potassium or a salt thereof, and basic fibroblast growth factor,
wherein the medium is obtained by adding the medium supplement to a neuronal culture medium, and
wherein in said enhanced synaptogenesis neuron model the number of synapses per neuron unit is increased and the neurotransmitter release per synapse is increased over the figures for primary neurons cultured in said neuronal culture medium not containing said medium supplement.

9. The use of claim 8, wherein the neuronal culture medium comprises neurotrophin-3 at a final concentration of 1 to 500 ng/mL, potassium or a salt thereof at a final concentration of 1 to 50 mM, and/or basic fibroblast growth factor at a final concentration of 1 to 100 ng/mL.

10. The use of claim 9, wherein the neuronal culture medium comprises neurotrophin-3 at a final concentration of 50 ng/mL, potassium or a salt thereof at a final concentration of 25 mM, and/or basic fibroblast growth factor at a final concentration of 5 ng/mL.

## Patentansprüche

1. Verfahren zur Herstellung eines Neuronenmodells mit *in* vivo-ähnlicher und verstärkter Synaptogenese, das einen Schritt der Kultivierung primärer Neuronen *in vitro* unter Verwendung eines Mediums umfasst,
wobei das Medium durch Hinzufügen eines Zusatzes zu einem Neuronenkulturmedium erhalten wird,
wobei der Zusatz eine Kombination von Neurotrophin-3, Kalium oder einem Salz davon und basischem Fibroblastenwachstumsfaktor umfasst, und
wobei in dem Neuronenmodell mit verstärkter Synaptogenese die Anzahl an Synapsen pro Neuroneinheit erhöht ist und die Neurotransmitterfreisetzung pro Synapse erhöht ist im Vergleich zu den Zahlen für die in dem Neuronenkulturmedium, das nicht diesen Zusatz enthält, kultivierten primären Neuronen.

2. Verfahren nach Anspruch 1, wobei das Neuronenmodell mit *in* vivo-ähnlicher und verstärkter Synaptogenese die Heldsche Kelch-Synapse ist, wenn das Gemisch von primären Neutronen aus dem ventralen Nucleus cochlearis und dem Nucleus medialis corporis trapezoidei als primäre Neuronen kultiviert werden.

3. Verfahren nach Anspruch 1 oder 2, wobei das Neuronenkulturmedium Neurotrophin-3 in einer Endkonzentration von 1 bis 500 ng/ml, Kalium oder ein Salz davon in einer Endkonzentration von 1 bis 50 mM und/oder basischen Fibroblastenwachstumsfaktor in einer Endkonzentration von 1 bis 100 ng/ml umfasst.

4. Verfahren nach Anspruch 3, wobei das Neuronenkulturmedium Neurotrophin-3 in einer Endkonzentration von 50 ng/ml, Kalium oder ein Salz davon in einer Endkonzentration von 25 mM und/oder basischen Fibroblastenwachstumsfaktor in einer Endkonzentration von 5 ng/ml umfasst.

5. Neuronenmodell mit *in* vivo-ähnlicher und verstärkter Synaptogenese, umfassend primäre Neuronen, die *in vitro* in einem Medium kultiviert werden,
wobei das Medium durch Hinzufügen eines Zusatzes zu einem Neuronenkulturmedium erhalten wird,
wobei der Zusatz eine Kombination von Neurotrophin-3, Kalium oder einem Salz davon und basischem Fibroblastenwachstumsfaktor umfasst, und
wobei in dem Neuronenmodell mit verstärkter Synaptogenese die Anzahl an Synapsen pro Neuroneinheit erhöht ist und die Neurotransmitterfreisetzung pro Synapse im Vergleich zu den Zahlen für die in dem Neuronenkulturmedium, das nicht diesen Zusatz enthält, kultivierten primären Neuronen.

6. Neuronenmodell mit *in* vivo-ähnlicher und verstärkter Synaptogenese nach Anspruch 5, wobei das Neuronenkulturmedium Neurotrophin-3 in einer Endkonzentration von 1 bis 500 ng/ml, Kalium oder ein Salz davon in einer Endkonzentration von 1 bis 50 mM und/oder basischen Fibroblastenwachstumsfaktor in einer Endkonzentration von 1 bis 100 ng/ml umfasst.

7. Neuronenmodell mit *in* vivo-ähnlicher und verstärkter Synaptogenese nach Anspruch 6, wobei das Neuronenkulturmedium Neurotrophin-3 in einer Endkonzentration von 50 ng/ml, Kalium oder ein Salz davon in einer Endkonzentration von 25 mM und/oder basischen Fibroblastenwachstumsfaktor in einer Endkonzentration von 5 ng/ml umfasst.

8. Verwendung eines Mediumzusatzes oder eines Mediums, das den Mediumzusatz umfasst, zur Herstellung eines Neuronenmodells mit *in* vivo-ähnlicher und verstärkter Synaptogenese bei der Zellkultur primärer Neuronen,
wobei der Mediumzusatz eine Kombination von Neurotrophin-3, Kalium oder einem Salz davon und basischem Fibroblastenwachstumsfaktor umfasst,
wobei das Medium durch Hinzufügen des Mediumzusatzes zu einem Neuronenkulturmedium erhalten wird, und
wobei in dem Neuronenmodell mit verstärkter Synaptogenese die Anzahl an Synapsen pro Neuroneinheit erhöht ist und die Neurotransmitterfreisetzung pro Synapse erhöht ist im Vergleich zu den Zahlen für die in dem Neuronenkulturmedium, das nicht diesen Mediumzusatz enthält, kultivierten primären Neuronen erhöht ist.

9. Verwendung nach Anspruch 8, wobei das Neuronenkulturmedium Neurotrophin-3 in einer Endkonzentration von 1 bis 500 ng/ml, Kalium oder ein Salz davon in einer Endkonzentration von 1 bis 50 mM und/oder basischen Fibroblastenwachstumsfaktor in einer Endkonzentration von 1 bis 100 ng/ml umfasst.

10. Verwendung nach Anspruch 9, wobei das Neuronenkulturmedium Neurotrophin-3 in einer Endkonzentration von 50 ng/ml, Kalium oder ein Salz davon in einer Endkonzentration von 25 mM und/oder basischen Fibroblastenwachstumsfaktor in einer Endkonzentration von 5 ng/ml umfasst.

## Revendications

1. Procédé de production d'un modèle neuronal de synaptogenèse semblable aux conditions *in vivo* et amélioré, qui comprend une étape de culture de neurones primaires *in vitro* en utilisant un milieu,
où le milieu est obtenu en ajoutant un supplément à un milieu de culture neuronale,
où le supplément comprend une combinaison de neurotrophine-3, de potassium ou d'un sel de celui-ci, et d'un facteur de croissance des fibroblastes basique, et
où dans ledit modèle neuronal de synaptogenèse amélioré, le nombre de synapses par unité neuronale est augmenté et la libération de neurotransmetteurs par synapse est augmentée par rapport aux caractéristiques de neurones primaires cultivés dans ledit milieu de culture neuronale ne contenant pas ledit supplément.

2. Procédé selon la revendication 1, où le modèle neuronal de synaptogenèse semblable aux conditions *in vivo* et amélioré est une synapse de type calice de Held, lorsque le mélange de neurones primaires issus de noyau cochléaire ventral et de noyau médian du corps trapézoïde est cultivé au titre de neurones primaires.

3. Procédé selon la revendication 1 ou 2, où le milieu de culture neuronale comprend de la neutrophine-3 à une concentration finale comprise entre 1 et 500 ng/mL, du potassium ou un sel de celui-ci à une concentration finale comprise entre 1 et 50 mM, et/ou un facteur de croissance des fibroblastes basique à une concentration finale comprise entre 1 et 100 ng/mL.

4. Procédé selon la revendication 3, où le milieu de culture neuronale comprend de la neutrophine-3 à une concentration finale de 50 ng/mL, du potassium ou un sel de celui-ci à une concentration finale de 25 mM, et/ou un facteur de croissance des fibroblastes basique à une concentration finale de 5 ng/mL.

5. Modèle neuronal de synaptogenèse semblable aux conditions *in vivo* et amélioré comprenant des neurones primaires cultivés *in vitro* dans un milieu,
où le milieu est obtenu par ajout d'un supplément à un milieu de culture neuronale, où le supplément comprend une combinaison de neutrophine-3, de potassium ou d'un sel de celui-ci, et d'un facteur de croissance des fibroblastes basique, et
où dans ledit modèle neuronal de synaptogenèse amélioré, le nombre de synapses par unité neuronale est augmenté et la libération de neurotransmetteurs par synapse est augmentée par rapport aux caractéristiques de neurones primaires cultivés dans ledit milieu de culture neuronale ne contenant pas ledit supplément.

6. Modèle neuronal de synaptogenèse semblable aux conditions *in vivo* et amélioré selon la revendication 5, où le milieu de culture neuronale comprend de la neutrophine-3 à une concentration finale comprise entre 1 et 500 ng/mL, du potassium ou un sel de celui-ci à une concentration finale comprise entre 1 et 50 mM, et/ou un facteur de croissance des fibroblastes basique à une concentration finale comprise entre 1 et 100 ng/mL.

7. Modèle neuronal de synaptogenèse semblable aux conditions *in vivo* et amélioré selon la revendication 6, où le milieu de culture neuronale comprend de la neutrophine-3 à une concentration finale de 50 ng/mL, du potassium ou un sel de celui-ci à une concentration finale de 25 mM, et/ou un facteur de croissance des fibroblastes basique à une concentration finale de 5 ng/mL.

8. Utilisation d'un supplément de milieu ou d'un milieu comprenant ledit supplément de milieu dans la production d'un modèle neuronal de synaptogenèse semblable aux conditions *in vivo* et amélioré dans une culture de cellules neuronales primaires,
où le supplément de milieu comprend une combinaison de neurotrophine-3, de potassium ou d'un sel de celui-ci, et d'un facteur de croissance des fibroblastes basique, où le milieu est obtenu en ajoutant le supplément de milieu à un milieu de culture neuronale, et
où dans ledit modèle neuronal de synaptogenèse amélioré, le nombre de synapses par unité neuronale est augmenté et la libération de neurotransmetteurs par synapse est augmentée par rapport aux caractéristiques de neurones primaires cultivés dans ledit milieu de culture neuronale ne contenant pas ledit supplément de milieu.

9. Utilisation selon la revendication 8, où le milieu de culture neuronale comprend de la neutrophine-3 à une concentration finale comprise entre 1 et 500 ng/mL, du potassium ou un sel de celui-ci à une concentration finale comprise entre 1 et 50 mM, et/ou un facteur de croissance des fibroblastes basique à une concentration finale comprise entre 1 et 100 ng/mL.

10. Utilisation selon la revendication 9, où le milieu de culture neuronale comprend de la neutrophine-3 à une concentration finale de 50 ng/mL, du potassium ou un sel de celui-ci à une concentration finale de 25 mM, et/ou un facteur de croissance des fibroblastes basique à une concentration finale de 5 ng/mL.
